(19) Europäisches Patentamt
European Patent Office
Office européen des brevets

(11) **EP 2 382 442 B1**

(12) **EUROPÄISCHE PATENTSCHRIFT**

(45) Veröffentlichungstag und Bekanntmachung des Hinweises auf die Patenterteilung:
**07.06.2017 Patentblatt 2017/23**

(51) Int Cl.:
**G01B 11/25** (2006.01)   **G01B 21/04** (2006.01)

(21) Anmeldenummer: **09802145.4**

(22) Anmeldetag: **23.12.2009**

(86) Internationale Anmeldenummer:
**PCT/EP2009/067861**

(87) Internationale Veröffentlichungsnummer:
**WO 2010/072816 (01.07.2010 Gazette 2010/26)**

(54) **VERFAHREN ZUR 3D-VERMESSUNG DER OBERFLÄCHE EINES OBJEKTS, INSBESONDERE FÜR ZAHNMEDIZINISCHE ZWECKE**

METHOD FOR 3D MEASUREMENT OF THE SURFACE OF AN OBJECT, IN PARTICULAR FOR DENTAL PURPOSES

PROCÉDÉ DE MESURE EN TROIS DIMENSIONS DE LA SURFACE D'UN OBJET, EN PARTICULIER POUR L'APPLICATION EN STOMATOLOGIE

(84) Benannte Vertragsstaaten:
**AT CH DE FR GB LI**

(30) Priorität: **24.12.2008 DE 102008055158**

(43) Veröffentlichungstag der Anmeldung:
**02.11.2011 Patentblatt 2011/44**

(73) Patentinhaber: **Sirona Dental Systems GmbH
64625 Bensheim (DE)**

(72) Erfinder:
• **SCHWOTZER, Axel
64521 Groß-Gerau (DE)**
• **KLEIN, Konrad
69121 Heidelberg (DE)**

(74) Vertreter: **Sommer, Peter
PRIO Patentanwälte
Augustaanlage 22
68165 Mannheim (DE)**

(56) Entgegenhaltungen:
**EP-A2- 0 968 687      US-A- 4 575 805
US-A- 5 175 601      US-A- 6 040 910
US-A1- 2007 115 484    US-A1- 2008 239 288**

EP 2 382 442 B1

**Beschreibung**

Technisches Gebiet

Die Erfindung betrifft ein Verfahren zur 3D-Vermessung der Oberfläche eines Objekts mittels optischer Doppeltriangulation unter Verwendung des Phasenschiebeverfahrens, insbesondere ein Verfahren zur Verbesserung der Messgenauigkeit einer nach dem Prinzip der Doppeltriangulation unter Verwendung des Phasenschiebeverfahrens arbeitenden 3D-Kamera, insbesondere für zahnmedizinische Zwecke und bezieht sich auf die Verbesserung der Qualität der ausgewerteten 3D-Messdatensätze.

Stand der Technik

Aus der US 4 575 805 ist ein Verfahren zur optischen 3D-Vermessung von Objekten, insbesondere von Zähnen, bekannt, welches das Triangulationsverfahren nutzt. Für das dort verwendete Phasenschiebeverfahren ist ein Algorithmus zur Berechnung des Phasenbildes aus mindestens drei Bildern eines auf ein Objekt projizierten Musters aus der US 6 040 910 bekannt.

Aus der EP 0 968 687 A2 ist eine Messkamera zur Erfassung von Oberflächenstrukturen, insbesondere für zahnmedizinische Zwecke bekannt, mit der in enger zeitlicher Reihenfolge mindestens zwei 3D-Vermessungen desselben Aufnahmeobjekts durchgeführt werden, wobei zwischen den beiden Vermessungen der Triangulationswinkel geändert wird. Dabei wird die Differenz der Messwerte verwendet, um den Eindeutigkeitsbereich der ersten Aufnahme zu vergrößern.

In der US 5,175,601 A ist eine Vorrichtung und ein Verfahren zur dreidimensionalen Vermessung von Oberflächen offenbart. Das Objekt wird mit einem Streifenmuster beleuchtet, wobei das projizierte Streifenmuster durch zwei Kameras aus zwei unterschiedlichen Raumwinkeln aufgenommen wird. Aus den beiden Aufnahmen der beiden Kameras wird dann anschlie-βend unter Verwendung des Triangulationsverfahrens ein dreidimensionales Bild erzeugt. Zur Lösung eines Eindeutigkeitsproblems wird ein Phaseschiebeverfahren verwendet.

In der US 2008/0239288 A1 eine Vorrichtung und ein Verfahren zur dreidimensionalen Formvermessung mit einer Stereo-Moire-Technik offenbart. Ein gestreiftes Muster wird mittels eines digitalen Projektors auf das Objekt projiziert und mittels einer ersten und der zweiten Kamera aus zwei verschiedenen Richtungen aufgenommen. In einem ersten Schritt des Verfahrens wird das phasenverschobene Streifenmuster auf das Objekt reduziert. In einem zweiten Schritt des Verfahrens werden zeitversetzt jeweils vier Aufnahmen mit jeder der Kamera aufgenommen. Aus den vier Aufnahmen wird die Phasenlage der Moire-Technik bestimmt. Im dritten Schritt des Verfahrens werden sich entsprechende Messpunkte in Aufnahmen der beiden Kameras festgestellt.

In der US 2007/0115484 A1 ist ein optisches Messverfahren offenbart, bei dem die Intensität eines projizierten Streifenmusters sinusförmig moduliert wird. Das Beleuchtungssystem zur Erzeugung des Streifenmusters kann mindestens drei Streifenmuster zeitversetzt auf das Objekt projizieren, die in ihrer Phase verschoben sind.

Die Messgenauigkeit der Kamera bei der Festlegung des Eindeutigkeitsbereichs hängt von der Reproduzierbarkeit der Änderung des Triangulationswinkels und damit von der Genauigkeit, mit welcher der Triangulationswinkel bekannt ist, ab. Bei einer vollständigen Reproduzierbarkeit des zweiten Triangulationswinkels kann die Messkamera durch Kalibrieren eine hohe Messgenauigkeit erzielen. Bei einer eingeschränkten Reproduzierbarkeit können die Abweichungen des zweiten Triangulationswinkels von dem vorgegebenen, zur Festlegung des Eindeutigkeitsbereichs herangezogenen Triangulationswinkel zu einer unzutreffenden Zuordnung des Eindeutigkeitsbereichs führen und somit ein fehlerhafter Höhenwert entstehen. Im Stand der Technik wird daher versucht, die Reproduzierbarkeit möglichst gut zu gewährleisten.

Weiterhin wurde in Kauf genommen, dass die zweite Meßaufnahme wegen möglicher Ungenauigkeiten nur zur Festlegung des Eindeutigkeitsbereichs herangezogen werden kann, nicht jedoch als eigenständige Messaufnahme verwertbar ist.

Der Erfindung liegt die Aufgabe zugrunde, die sich aus der mangelhaften Reproduzierbarkeit des zweiten Triangulationswinkels ergebenden Fehler bei der Bestimmung der Höhenwerte weiter zu verringern. Darüber hinaus ist es wünschenswert, wenn auch die zweite Messaufnahme zur Bestimmung eines 3D-Datensatzes nutzbar ist.

Darstellung der Erfindung

Diese Aufgabe wird durch ein Verfahren mit den Merkmalen des unabhängigen Verfahrensanspruchs 1 gelöst. Vorteilhafte Weiterbildungen ergeben sich aus den abhängigen Ansprüchen.

Erfindungsgemäß wird ein 3D-Datensatz eines Objekts, insbesondere eines oder mehrerer Zähne, mittels optischer Doppeltriangulation unter Verwendung des Phasenschiebeverfahrens erstellt. Dazu werden mindestens zwei 3D-Vermessungen desselben Objekts mit unterschiedlichen Trianguluationswinkeln durchgeführt, die für jeden Triangulationswinkel wenigstens ein Phasenbild mit mehreren Bildpunkten mit den Koordinaten ergeben, wobei der erste Triangulationswinkel bekannt und der zweite Triangulationswinkel zumindest näherungsweise bekannt ist. Jedes Phasenbild weist einer Eindeutigkeitsbereich auf, der unter anderem vom jeweiligen Triangulationswinkel abhängt.

Für jeden Bildpunkt des ersten Phasenbildes wird unter Verwendung des zweiten Phasenbildes eine Wellenzahl bestimmt, deren ganzzahliger Anteil der Ordnung des Eindeutigkeitsbereichs entspricht, in dem sich der jeweilige Bildpunkt befindet. Diese Wellenzahl wird dahingehend optimiert, dass zumindest für eine Stichprobe von m Bildpunkten ein nicht ganzzahlige Anteil der Wellenzahl minimiert wird.

Die optimierte Wellenzahl kann dazu verwendet werden, ein Höhenbild des Objekts zu bestimmen oder das Verhältnis von Skalierungsfaktoren, welche die Phasenbilder auf metrische Höhenwerte abbilden, und einen Drift zwischen den zwei Phasenbildern festzulegen und als Startwerte für die Optimierung eines weiteren Höhenbildes zu verwenden oder dazu, ein zweites metrisches Höhenbild aus dem zweiten Phasenbild zu erzeugen.

Aufgrund einer mangelnden Reproduzierbarkeit des zweiten Triangulationswinkels kann ein nur näherungsweise bekannter zweiter Triangulationswinkel vorliegen. So kann zur Erzeugung eines zweiten Triangulationswinkels eine Blende den Schwerpunkt des Beleuchtungsstrahls verschoben werden. Bei einer mechanischen Realisierung kann die Reproduzierbarkeit fehlerbehaftet sein.

Die Phasenbilder können, wie es aus dem Stand der Technik bekannt ist, jeweils aus mehreren Bildern eines auf das Objekt projizierten periodischen Musters gebildet werden, wobei das Muster eine bekannte, jeweils gegenüber dem vorherigen Bild verschobene Phasenlage hat.

Der Zusammenhang zwischen einer Phasenverschiebung des Musters gegenüber einer Referenz und dem entsprechenden Phasenbild ist durch eine eindeutige, periodische Funktion gegeben.

Ist der der Messbereich in der Höhe größer als der Eindeutigkeitsbereich, so ist die Abbildung der Phasenbilder auf metrische Höhenwerte mehrdeutig. Es ist allerdings aus dem Stand der Technik bekannt, dass aufgrund der Differenz zwischen den zwei Phasenbilder in einem Bildpunkt diesem Bildpunkt eine Ordnung des Eindeutigkeitsbereichs zugeordnet werden kann, in dem er sich befindet, um damit die Mehrdeutigkeit aufzulösen.

Vorteilhafterweise hat das auf das Objekt projizierte Muster einen sinusförmigen Helligkeitsverlauf mit bekannter, verschiebbarer Phasenlage. Dadurch lassen sich geläufige Auswertealgorithmen verwenden.

Vorteilhafterweise ist der erster Skalierungsfaktor genau bekannt, der das erste Phasenbild auf metrische Höhenwerte h abbildet und ist der zweiter Skalierungsfaktor zumindest näherungsweise bekannt, der das zweite Phasenbild auf metrische Höhenwerte h abbildet.

Diese Skalierungsfaktoren können mittels einer der Vermessung des Objekts vorangehenden Kalibration ermittelt werden und sind zum Tangens des jeweiligen Triangulationswinkels proportional.

Wenn für das aufgenommene Phasenbildpaar die ganzzahligen Anteile der Wellenzahlen für mindestens zwei Bilpunkte $B_i$ verschieden sind, kann die Optimierung der Wellenzahl vorteilhafterweise dahingehend erfolgen, dass zumindest für eine Stichprobe von m Bildpunkten ein nicht ganzzahliger Anteil der Wellenzahl durch Änderung des Verhältnisses der Skalierungsfaktoren über den zumindest näherungsweise bekannten Skalierungsfaktor und durch Änderung eines Drifts der Phasenlage des Musters zwischen den zwei Phasenbildern minimiert wird.

Dieses vollständige Optimierungsverfahren ermöglicht es Fehler in der Zuordnung der Ordnung des Eindeutigkeitsbereichs eines Punktes zu verringern und damit eine verbesserte Bestimmung des Höhenbildes vorzunehmen.

Dieses Optimierungsverfahren ermöglicht auch eine verbesserte Bestimmung des zweiten Triangulationswinkels. So kann die Maßhaltigkeit der zweiten Aufnahme auch ohne eine weitere Kalibrierung mittels eines Kalibrierungskörpers hergestellt werden. Es handelt sich also um ein Selbstkalibrationsverfahren.

Falls für das aufgenommene Phasenbildpaar die ganzzahligen Anteile der Wellenzahl für alle Bildpunkte gleich sind, kann die Optimierung der Wellenzahl vorteilhafterweise dahingehend erfolgen, dass zumindest für eine Stichprobe von m Bildpunkte der nicht ganzzahlige Anteil der Wellenzahl durch Änderung des Drifts zwischen den beiden Phasenbildern minimiert wird.

Mit diesem Teiloptimierungsverfahren kann zumindest der Fehler, der aufgrund einer Driftbewegung des Musters die Wellenzahl verfälscht und damit zu fehlerhaften Höhenwerten führt, korrigiert werden.

Für die m-elementige Stichprobe aus den Phasenbildern können vorteilhafterweise anhand der Messdatenqualität und eines geeigneten Ausreißer-Erkennungs-Verfahrens fehlerhafte Bildpunkte ausgeschlossen werden, wobei die Bildpunkte der Stichprobe insbesondere innerhalb einer erwarteten Verteilung bezüglich des nicht ganzzahligen Anteils der Verteilung liegen.

Die geeignete Auswahl der Stichprobe umfasst Messwerte aus dem mittleren Bildbereich, der in der Regel innerhalb des Messbereichs liegt, und nicht aus dem Randbereich der Bilder. Dies führt in Kombination mit dem Ausschluß fehlerhafter Bildpunkte zu besseren Ergebnissen des Optimierungsverfahrens.

Zur Ausreißer-Erkennung kann beispielsweise die Modulationsamplitude der projizierten Sinus-Muster-Sequenz im Phasenbild verwendet werden, indem die tatsächliche Verteilung der Amplituden mit einer erwarteten Verteilung verglichen wird.

Vorteilhafterweise können die Aufnahme der Bilder für das erste und das zweite Phasenbild in enger zeitlicher Reihenfolge erfolgen. Dies hat den Vorteil, dass die Position der Kamera vor allem bei der Verwendung einer handgehaltenen Kamera zwischen den Aufnahmen gar nicht oder nur geringfügig geändert wird.

Zur Erzeugung eines verbesserten zweiten Höhenbildes aus dem zweiten Phasenbild können vorteilhafterweise in einem Optimierungsverfahren unter Verwendung des ersten aus der Optimierung erhaltenen Höhenbildes sowohl der nur ungenau bekannte Skalierungsfaktor des zweiten Phasenbildes, als auch eine Positionsänderung der Kamera zwischen dem zweiten Höhenbild und dem ersten, unter Verwendung der optimierten Wellenzahl erhaltenen Höhenbild berücksichtigt werden, wobei eine Positionsänderung der Kamera als eine Rotations- und eine Translationsbewegung angenommen wird.

Dies hat den Vorteil, dass das zweite Phasenbild trotz der Unsicherheit des tatsächlichen Triangulationswinkels auch als vollwertige Messaufnahme zur Erstellung eines Höhenbildes verwendet werden kann.

Kurzbeschreibung der Zeichnungen

Ein Ausführungsbeispiel der Erfindung wird anhand der Zeichnung erläutert. Es zeigt:

Fig. 1    ein grafisches Schema zur Verdeutlichung des erfinderischen Verfahrens mit einer 3D-Kamera und einer Bildverarbeitungseinheit,

Fig. 2    den Zusammenhang zwischen der Phasenverschiebung und dem Phasenbild,

Fig. 3    den Zusammenhang zwischen den Höhenwerten und den Phasenbildern sowie den idealisierten Zusammenhang zwischen den Höhenwerten und der Wellenzahl,

Fig. 4    den Zusammenhang zwischen den Höhenwerten und der Wellenzahl für eine reale 3D-Vermessung. Ausführungsbeispiel

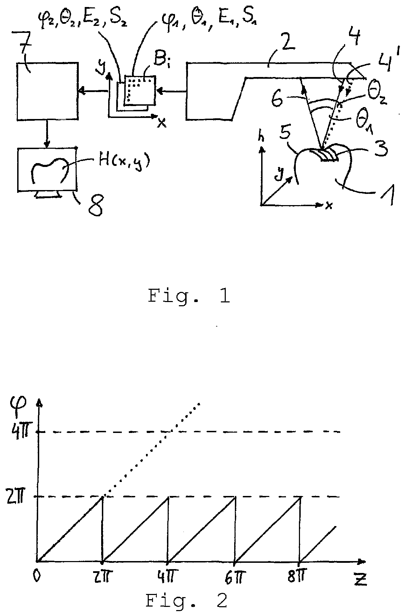

Wie in Fig. 1 schematisch dargestellt, wird bei einer 3D-Vermessung eines Objekts 1 mittels Doppeltriangulation unter Verwendung des Phasenschiebeverfahrens mit einer 3D-Kamera 2 ein periodisches Muster 3 mit einer bekannten Phaselage von einem Beleuchtungsstrahl 4 oder 4' auf einen Teil der Oberfläche 5 des Objekts 1 abgebildet.

In einer Beobachtungsrichtung 6, welche mit dem Beleuchtungsstrahl 4 einen Triangulationswinkel $\theta 1$ und mit dem Beleuchtungsstrahl 4' einen Triangulationswinkel $\theta 2$ einschließt, wird für Bildpunkte B_mit den Koordinaten $x_i$ und $y_i$ eine von der Oberfläche 5 des Objekts 1 mit dem abgebildeten Muster 3 gestreute Intensität aufgenommen. Wenn der Triangulationswinkel $\theta 1$ bekannt und der Triangulationswinkel $\theta 2$ nur näherungsweise bekannt ist, etwa weil der Winkel $\theta 2$ nicht genau reproduzierbar ist, so kann die zweite Aufnahme nur eingeschränkt verwendet werden, da sie fehlerbehaftet ist.

Aus mehreren für jeweils einen Triangulationswinkel $\theta 1$ oder $\theta 2$ aufgenommenen Intensitäten für verschiedene Phasenlagen des Musters kann für die einzelnen Bildpunkte $B_i$ eine Phase ermittelt werden und die Gesamtheit dieser Phasen wird nachfolgend als Rohphasenbild p1(x,y) für unter dem Triangulationswinkel $\theta 1$ aufgenommene Bilder und Rohphasenbild p2(x,y) für unter dem zweiten Triangulationswinkel $\theta 2$ aufgenommene Bilder bezeichnet.

Weiterhin kann es zwei durch Kalibrieren bekannte Referenzbilder r1(x,y) und r2(x,y) geben, die jeweils ein unter Verwendung des entsprechenden Triangulationswinkels aufgenommenes Phasenbild des Musters projiziert auf eine zur Kamerablickachse senkrechte ebene Fläche sind. Diese Referenzbilder r1 und r2 können vom Phasenbild p1(x,y) bzw. p2(x,y) abgezogen werden, wobei diese Differenz einer Phasenverschiebung der Phase des Phasenbildes p1(x,y) bzw. p2(x,y) relativ zur Phase des Referenzbildes r1(x,y) bzw. r2(x,y) an einem Bildpunkt $B_i$ entspricht und proportional zur Höhe der Oberfläche des Objekts an diesem Bildpunkt $B_i$ ist.

Da die Phase der aufgenommenen Rohphasenbilder p1(x,y) und p2(x,y) allerdings nur innerhalb eines Bereichs $[0,2\pi)$ eindeutig sind, kann die Differenz zwischen Rohphasenbild p1(x,y) bzw. p2(x,y) und Referenzbild r1(x,y) bzw. r2(x,y) daher auch auf den Bereich $[0,2\pi)$ beschränkt werden und wird nachfolgend als Phasenbild $\varphi 1(x,y)$ bzw. $\varphi 2(x,y)$ oder auch orthogonalisiertes Phasenbild bezeichnet.

Die Abbildung der Phasenbilder $\varphi 1$ und $\varphi 2$ auf Höhenbilder H1 und H2 erfolgt über Skalierungsfaktoren S1 und S2, wobei diese Skalierungsfaktoren durch eine einer Messung vorangegangene Kalibrierung unter dem jeweiligen Triangulationswinkel $\theta 1$ oder $\theta 2$ bekannt sind. Da der Triangulationswinkel $\theta 2$ nur näherungsweise reproduzierbar ist, ist der Skalierungsfaktor S2 fehlerbehaftet.

Eine Zuordnung eines Höhenwerts h zu einer Phase der Phasenbilder $\varphi 1(x,y)$ und $\varphi 2(x,y)$ kann nur innerhalb eines jeweiligen Eindeutigkeitsbereichs E1 bzw. E2 eindeutig vorgenommen werden, der sowohl vom Triangulationswinkel $\theta 1$, $\theta 2$ als auch von der Periode des Musters abhängt.

Wenn der zu vermessende Höhenbereich größer als der Eindeutigkeitsbereich E1 bzw. E2 ist, so ist die Zuordnung von Höhenwerten h zum jeweiligen Phasenbild $\varphi 1$ bzw. $\varphi 2$ mehrdeutig.

Das Höhenbild wird in einer Bildverarbeitungseinheit 7 ermittelt und kann in einer Ausgabeeinheit 8 ausgegeben werden. Die Ermittlung des Höhenbildes wird in den Fig. 2 bis 4 genauer dargestellt.

In Fig. 2 ist der Zusammenhang zwischen einer realen, nicht auf den Bereich [0,2n) beschränkten Phasenverschiebung z, die die Verschiebung zwischen der Phase des auf das Objekt abgebildeten Musters und der Phase der Referenzbilder r1(x,y) bzw. r2(x,y) an einem Bildpunkt $B_i$ angibt, und den Phasenbildern φ1(x,y) und φ2(x,y) dargestellt, der die Form eines Sägezahns mit einer Periodenlänge von 2n und einer Amplitude von ebenfalls 2n hat. Dieser Zusammenhang ergibt sich aus der Mehrdeutigkeit der Phasenbilder φ1(x,y) und φ2(x,y).

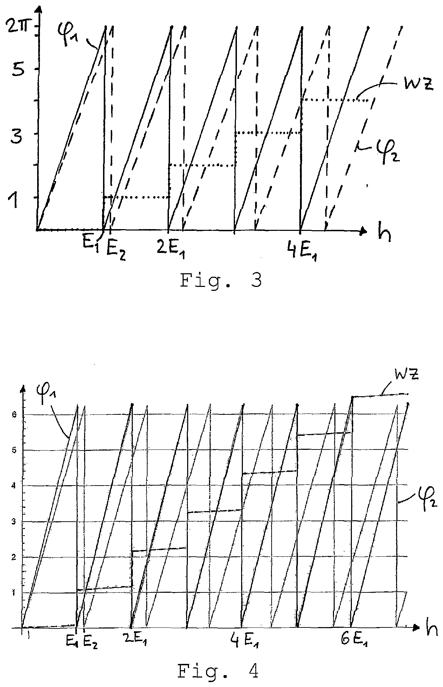

Fig. 3 zeigt den sich aus der Mehrdeutigkeit der Phasenbilder φ1(x,y) und φ2(x,y) ergebenden Zusammenhang zwischen den Phasen eines jeweiligen Phasenbildes φ1(x,y) bzw. φ2(x,y) und den Höhenwerten h. Die Periodenlänge des jeweiligen Sägezahns entspricht dem jeweiligen Eindeutigkeitsbereich E1 oder E2.

Es ist zu erkennen, dass die Differenz der Phasenbilder φ1(x,y) und φ2(x,y) mit steigenden Höhenwerten h ebenfalls größer wird. Diese Differenz der Phasenbilder φ(x,y), φ2(x,y) erlaubt einen Rückschluss darauf, in welcher Ordnung n eine in einem Bildpunkt $B_i$ gemessene Phase eines Phasenbildes φ1($x_i,y_i$) liegt. Dies ist aus der EP 0 968 687 A2 bekannt.

Unter Verwendung einer skalierten Differenz der Phasenbilder φ1 und φ2 kann zu jedem Bildpunkt eine Wellenzahl wz berechnet werden, die der Ordnung des Eindeutigkeitsbereichs des Bildpunkts des ersten Phasenbildes φ1 entspricht. Die Formel hierzu lautet:

$$wz = ((r*\varphi1 - \varphi2) \bmod (2\pi))/(2\pi*(1-r)),$$

wobei r das Verhältnis zwischen den zwei Skalierungsfaktoren S1 und S2 angibt. Bei fehlerfreier Messung der Phasenbilder φ1, φ2 und mit fehlerfreien Skalierungsfaktoren S1 und S2 ist die Wellenzahl stets ganzzahlig. Der sich ergebende treppenförmige Verlauf dieser Wellenzahl als Funktion der Höhenwerte h, der ebenfalls in Fig. 3 dargestellt ist, entspricht also einem idealisierten Verlauf.

Fig. 4 zeigt einen realen Verlauf der Wellenzahl, die unter Verwendung eines fehlerbehafteten Skalierungsfaktors S2 ermittelt wurde. Der Fehler des Skalierungsfaktors führt zu einem mit dem Höhenwert h stetig wachsenden Fehler der Wellenzahl. Diese nimmt also nicht mehr nur ganzzahlige Werte an, sondern hat einen mit dem Höhenwert h stetig steigenden nicht ganzzahligen Anteil, so dass die Stufen des treppenförmigen Verlaufs des Zusammenhangs zwischen Höhenwerten h und Wellenzahl wz nicht mehr eben sind, sondern eine von Null verschiedene Steigung haben. In Fig. 4 sind waagerechte Hilfslinien eingezeichnet, um den schrägen Anstieg der einzelnen Stufen zu verdeutlichen.

Auch eine Driftbewegung des Musters zwischen den Vermessungen der zwei Phasenbilder φ1, φ2 kann zu einem Fehler bei der Bestimmung der Wellenzahl wz führen. Ein solcher Fehler führt zu einem konstanten Versatz des Verlaufs der Wellenzahl als Funktion der Höhe nach oben oder unten. Der in den Fig. 3, 4 dargestellte treppenförmige Verlauf würde durch einen solchen Fehler nach oben oder unten verschoben werden, am Verlauf der einzelnen Stufen selbst ändert ein solcher Fehler nichts.

Aus der Abweichung vom idealen treppenförmigen Verlauf der Wellenzahl, also aus dem nicht ganzzahligen Anteil der Wellenzahl kann der Fehler der Eingangsgrößen abgelesen werde und mithilfe eines Optimierungsverfahren kann diese Abweichung minimiert werden. Dazu kann unter Verwendung eines Fehlerquadrat-Minimierungsverfahrens der nicht ganzzahlige Anteil der Wellenzahl wz minimiert werden, indem bei einer Fehlerquadrat-Minimierungsaufgabe sowohl das Verhältnis r über den Skalierungsfaktor S2 als auch der Drift q zwischen den Phasenlagen des Musters in den zwei Phasenbildern φ1 und φ2 verändert werden. Hierzu müssen sich allerdings mindestens zwei Wellenzahlen wz der Phasenbilder φ1 und φ2 in ihrem ganzzahligen Anteil unterscheiden.

Sind die ganzzahligen Anteile aller Wellenzahlen wz der zwei Phasenbilder φ1 und φ2 gleich, so kann in einem Optimierungsverfahren der nicht ganzzahlige Anteil der Wellenzahlen durch Ändern des Drifts q zwischen den Phasenlagen des Musters in den zwei Phasenbildern φ1 und φ2 minimiert werden.

Die Höhenwerte h des Höhenbildes H1(x,y) können aus dem Phasenbild (φ1(x,y) ermittelt werden, indem für jeden Bildpunkt B_ zum Phasenbild φ1($x_i,y_i$) das 2π-fache der optimierten Wellenzahl wz($x_i,y_i$) addiert wird und diese Summe mit einem Skalierungsfaktor S1 multipliziert wird.

Die beschriebenen Optimierungsverfahren sind dazu geeignet, die Wellenzahl wz mit der nötigen Zuverlässigkeit zu bestimmen und die Mehrdeutigkeiten der Phasenbilder φ1 und φ2 sicher aufzulösen.

Aus dem zweiten Phasenbild φ2 kann nach einem weiteren Optimierungsverfahren ein zweites Höhenbild H2 des Objekts erzeugt werden. Unter Verwendung eines Fehlerquadrat-Minimierungsverfahrens kann hierfür die Differenz zwischen den zwei Höhenbilder H1 und H2 minimiert werden, indem sowohl der optimierte Skalierungsfaktor S2 weiter optimiert, als auch eine Positionsänderung der Kamera durch Rotation und Translation des ersten Höhenbildes H1 berücksichtigt wird.

EP 2 382 442 B1

Bezugszeichenliste

1 Objekt
2 3D-Kamera
3 Muster
4 Beleuchtungsstrahl
5 Oberfläche des Objekts
6 Beobachtungsrichtung
7 Bildverarbeitungseinheit
8 Ausgabeeinheit
$\theta$ Triangulationswinkel
$\varphi$ Phasenbild
$B_i$ Bildpunkt
S Skalierungsfaktor
E Eindeutigkeitsbereich
z Phasenverschiebung
h metrischer Höhenwert
H Höhenbildes
wz Wellenzahl

**Patentansprüche**

1. Verfahren zur 3D-Vermessung der Oberfläche eines Objekts mittels optischer Doppeltriangulation unter Verwendung des Phasenschiebeverfahrens, insbesondere für zahnmedizinische Zwecke,

   a. wobei mindestens zwei 3D-Vermessungen desselben Objekts (1) mit unterschiedlichen Triangluationswinkeln ($\theta1$, $\theta2$) durchgeführt werden, die für jeden Triangulationswinkel ($\theta1$, $\theta2$) wenigstens ein Phasenbild ($\varphi1$, $\varphi2$) mit mehreren Bildpunkten ($B_i$) mit den Koordinaten ($x_i$,$y_i$) ergeben, wobei das Phasenbild ($\varphi1$, $\varphi2$) aus mehreren Bildern eines auf ein Objekt (1) projizieren periodischen Musters (3) gebildet ist, das eine bekannte Phasenlage aufweist,
   b. wobei der erste Triangulationswinkel ($\theta1$) bekannt ist,
   c. wobei jedes Phasenbild ($\varphi1$, $\varphi2$) einen Eindeutigkeitsbereich (E1, E2) aufweist, der unter anderem vom Triangulationswinkel ($\theta1$, $\theta2$) abhängt, wobei eine Zuordnung eines Höhenwerts (h) zu einer Phase der Phasenbilder nur innerhalb eines jeweiligen Eindeutigkeitsbereichs (E1, E2) eindeutig vorgenommen werden kann, und
   d. wobei die Abbildung jedes Phasenbildes ($\varphi1$, $\varphi2$) mittels eines Skalierungsfaktors (S1,S2) auf metrisache Höhenwerte erfolgt,

   **dadurch gekennzeichnet, dass**

   e. der zweite Triangulationswinkel ($\theta2$) nur näherungsweise reproduzierbar ist und der zweite Skalierungsfaktor (S2) damit fehlerhaft ist,
   f. für jeden Bildpunkt ($B_i$) des ersten Phasenbildes $\varphi1$ unter Verwendung des zweiten Phasenbildes $\varphi2$ eine Wellenzahl (wz), nach der Formel :

$$wz=((r*\varphi1-\varphi2)\bmod(2\pi))/2\pi*(1-r)),$$

   bestimmt wird, wobei r das Verhältnis zwischen den zwei Skalierungsfaktoren S1 und S2 angibt, und wobei S1 der erste und S2 der zweite Skalierungsfaktor ist, deren ganzzahliger Anteil der Ordnung (n) des Eindeutigkeitsbereichs (E1) entspricht, in dem sich der jeweilige Bildpunkt ($B_i$) befindet, wobei der nicht ganzzahlige Anteil der Wellenzahl (wz) mit einem Höhenwert (h) stetig steigt und damit Fehler des fehlerbehafteten zweiten Skalierungsfaktors (S2) abbildet
   g. und dass eine Optimierung der Wellenzahl (wz) dahingehend erfolgt, dass, zumindest für eine Stichprobe

von m Bildpunkten ($B_i$) der nicht ganzzahlige Anteil der Wellenzahl durch Änderung des Verhältnisses der Skalierungsfaktoren (S1, S2) und durch Änderung eines Drifts (9) der Phasenlage des Musters (3) zwischen den beiden Phasenbildern ($\varphi$1, $\varphi$2) minimiert wird,

h. wobei die optimierte Wellenzahl wie folgt verwendet wird:

h.1. zur Bestimmung eines ersten Höhenbildes (H1) des Objekts, indem für jeden Bildpunkt ($B_i$) die Summe des Phasenbildes ($\varphi$1) an diesem Bildpunkt ($B_i$) mit dem $2\pi$-fachen der zugehörigen Wellenzahl (wz) gebildet und die Summe mit dem Skalierungsfaktor (S1) multipliziert wird oder

h.2. zur Festlegung von einem Verhältnis (r) der Skalzerungsfaktoren (S1, S2), welche die Phasenbilder ($\varphi$1 $\varphi$2) auf metrische Höhenwerte (h) abbilden und eines Drifts (q) zwischen der Phasenlage des Musters (3) in den beiden Phasenbildern ($\varphi$1, $\varphi$2) als Startwerte für die Optimierung des nächsten Höhenbildes (H) der nächsten 3D-Vermessung des Objekts oder

h.3. zur Erzeugung eines zweiten metrischen Höhenbildes (H2) aus dem zweiten Phasenbild ($\varphi$2).

2. Verfahren nach Anspruch 1, **dadurch gekennzeichnet, dass** zur Durchführung des Phasenschiebeverfahrens ein Muster auf das Objekt projiziert wird, welches einen sinusförmigen Helligkeitsverlauf mit bekannter, verschiebbarer Phasenlage aufweist.

3. Verfahren nach einem der Ansprüche 1 oder 2, **dadurch gekennzeichnet, dass** der erste Skalierungsfaktor (S1) genau bekannt ist, der das erste Phasenbild ($\varphi$1) auf metrische Höhenwerte (h) abbildet und dass der zweite Skalierungsfaktor (S2) zumindest näherungsweise bekannt ist, der das zweite Phasenbild ($\varphi$1) auf metrische Höhenwerte (h) abbildet.

4. Verfahren nach einem der Ansprüche 1 bis 3, **dadurch gekennzeichnet, dass**, wenn für das aufgenommene Phasenbildpaar ($\varphi$1, $\varphi$2) für mindestens zwei Punkte ein ganzzahliger Anteil der Wellenzahl dieser Bilpunkte ($B_i$) verschieden ist, die Optimierung der Wellenzahl dahingehend erfolgt, dass zumindest für eine Stichprobe von m Bildpunkten ($B_i$) ein nicht ganzzahliger Anteil der Wellenzahl durch Änderung des Verhältnisses (r) der Skalierungsfaktoren (S1, S2) über den zumindest näherungsweise bekannten zweiten Skalierungsfaktor (S2) und durch Änderung des Drifts (q) des Musters (3) zwischen den beiden Phasenbildern ($\varphi$1, $\varphi$2) minimiert wird.

5. Verfahren nach einem der Ansprüche 1 bis 4, **dadurch gekennzeichnet, dass**, falls für das aufgenommene Phasenbildpaar ($\varphi$1, $\varphi$2) die ganzzahligen Anteile der Wellenzahl für alle Bildpunkte ($B_i$) gleich sind, die Optimierung der Wellenzahl dahingehend erfolgt, dass zumindest für eine Stichprobe von m Bildpunkten ($B_i$) der nicht ganzzahlige Anteil der Wellenzahl durch Änderung des Drifts (q) zwischen den beiden Phasenbildern ($\varphi$1, $\varphi$2) minimiert wird.

6. Verfahren nach einem der Ansprüche 1 bis 5, **dadurch gekennzeichnet, dass** für die m-elementige Stichprobe aus den Phasenbildern ($\varphi$1, $\varphi$2) anhand der Messdatenqualität und eines geeigneten Ausreißer-Erkennungs-Verfahrens fehlerhafte Bildpunkte ausgeschlossen werden, wobei die Bildpunkte der Stichprobe insbesondere innerhalb einer erwarteten Verteilung bezüglich des nicht ganzzahligen Anteils der Verteilung liegen.

7. Verfahren nach einem der Ansprüche 1 bis 6, **dadurch gekennzeichnet, dass** das die Aufnahme der Bilder für das erste und das zweite Phasenbild in enger zeitlicher Reihenfolge erfolgen.

8. Verfahren nach einem der Ansprüche 1 bis 7, **dadurch gekennzeichnet, dass** zur Erzeugung eines zweiten Höhenbildes (H2) aus dem zweiten Phasenbild ($\varphi$2) in einem Optimierungsverfahren unter Verwendung des ersten aus der Optimierung erhaltenen Höhenbildes (H1) sowohl der nur ungenau bekannte Skalierungsfaktor (S2), als auch eine Positionsänderung der Kamera zwischen den Phasenbildern ($\varphi$1) und ($\varphi$2) berücksichtigt werden, wobei als Positionsänderung der Kamera eine Rotations- und eine Tranlationsbewegung angenommen wird.

**Claims**

1. Method for 3-D measurement of the surface of an object by means of optical double triangulation using the phase shift method, especially for purposes of dentistry,

a. wherein at least two 3-D measurements of the same object (1) are performed with different triangulation angles ($\theta$1, $\theta$2) that, for each triangulation angle ($\theta$1, $\theta$2), yield at least one phase image ($\varphi$1, $\varphi$2) having multiple image points (Bi) with the coordinates (xi, yi), wherein the phase image ($\varphi$1, $\varphi$2) is formed from multiple images of a periodic pattern (3) projecting onto an object (1), which pattern (3) has a known phase position,

b. wherein the first triangulation angle ($\theta1$) is known,

c. wherein each phase image ($\varphi1$, $\varphi2$) has a uniqueness range (E1, E2) that, among other things, depends upon the triangulation angle ($\theta1$, $\theta2$), wherein an association of a height value (h) with a phase of the phase images may be made unambiguously only within a respective uniqueness range (E1, E2), and

d. wherein the mapping of each phase image ($\varphi1$, $\varphi2$) to metric height values takes place by means of a scaling factor (S1, S2),

**characterized in that**

e. the second triangulation angle ($\theta2$) is only approximately reproducible, and the second scaling factor (S2) is therefore incorrect,

f. for each image point (Bi) of the first phase image $\varphi1$, a wave number (wz) is determined using the second phase image $\varphi2$, according to the formula:

$$wz=((r*\varphi1-\varphi2)\bmod(2\pi))/2\pi*(1-r)),$$

wherein r indicates the ratio between the two scaling factors S1 and S2, and wherein S1 is the first scaling factor and S2 is the second scaling factor,

whose integral portion corresponds to the magnitude (n) of the uniqueness range (E1) in which the respective image point (Bi) is located, wherein the non-integral portion of the wave number (wz) increases steadily with a height value (h), and thereby maps errors of the erroneous second scaling factor (S2),

g. and **in that** an optimization of the wave number (wz) takes place to the effect that, at least for a sample of m image points (Bi), the non-integral portion of the wave number is minimized by changing the ratio of the scaling factors (S1, S2) and by changing a drift (9) of the phase position of the pattern (3) between the two phase images ($\varphi1$, $\varphi2$),

h. wherein the optimized wave number is used as follows:

h.1. to determine a first elevation image (H1) of the object, **in that**, for each image point (Bi), the sum of the phase image ($\varphi1$) at this image point (Bi) is formed with 2n times the associated wave number (wz), and the sum is multiplied by the scaling factor (S1), or

h.2. to establish a ratio (r) of the scaling factors (S1, S2) which map the phase images ($\varphi1$, $\varphi2$) to metric height values (h), and to establish a drift (q) between the phase position of the pattern (3) in the two phase images ($\varphi1$, $\varphi2$) as start values for the optimization of the next elevation image (H) of the next 3-D measurement of the object, or

h.3. to generate a second metric elevation image (H2) from the second phase image ($\varphi2$).

2. Method according to claim 1, **characterized in that** a pattern is projected onto the object in order to implement the phase shift method, which pattern has a sinusoidal brightness curve with known, displaceable phase position.

3. Method according to one of claims 1 or 2, **characterized in that** the first scaling factor (S1) is precisely known and maps the first phase image ($\varphi1$) to metric height values (h), and **in that** the second scaling factor (S2) is at least approximately known and maps the second phase image ($\varphi2$) to metric height values (h).

4. Method according to one of claims 1 through 3, **characterized in that**, for the acquired phase image pair ($\varphi1$, $\varphi2$) for at least two points, if an integral portion of the wave number of these image points (Bi) is different, the optimization of the wave number takes place to the effect that, for at least a sample of m image points (Bi), a non-integral portion of the wave number is minimized by changing the ratio (r) of the scaling factors (S1, S2), via the at least approximately known second scaling factor (S2), and by changing the drift (q) of the pattern (3) between the two phase images ($\varphi1$, $\varphi2$).

5. Method according to one of claims 1 through 4, **characterized in that**, for the acquired phase image pair ($\varphi1$, $\varphi2$), in the event that the integral portions of the wave number for all image points (Bi) are the same, the optimization of the wave number takes place to the effect that - for at least a sample of m image points (Bi) - the non-integral portion of the wave number is minimized by changing the drift (q) between the two phase images ($\varphi1$, $\varphi2$).

6. Method according to one of claims 1 through 5, **characterized in that**, for the m-element sample, incorrect image points are excluded from the phase images ($\varphi 1$, $\varphi 2$) using the measurement data quality and a suitable outlier detection method, wherein the image points of the sample lie, in particular, within an expected distribution with respect to the non-integral portion of the distribution.

7. Method according to one of claims 1 through 6, **characterized in that** the acquisition of the images for the first and second phase image takes place in rapid chronological order.

8. Method according to one of claims 1 through 7, **characterized in that**, to generate a second elevation image (H2) from the second phase image ($\varphi 2$) in an optimization method using the first elevation image (H1) obtained from the optimization, both the scaling factor (S2) (which is only imprecisely known) and a position change of the camera between the phase images ($\varphi 1$) and ($\varphi 2$) are considered, wherein a rotation and translation movement is assumed as a position change of the camera.

## Revendications

1. Procédé de mesure en trois dimensions de la surface d'un objet au moyen d'une double triangulation optique en utilisant un procédé de déphasage, en particulier à des fins médico-dentaires,

a. dans lequel au moins deux mesures en trois dimensions du même objet (1) sont réalisées avec des angles de triangulation différents (91, $\theta 2$), qui produisent, pour chaque angle de triangulation ($\theta 1$, $\theta 2$) au moins une image de phase ($\varphi 1$, $\varphi 2$) avec plusieurs pixels (Bi) avec les coordonnées (xi, yi), dans lequel l'image de phase ($\varphi 1$, $\varphi 2$) est formée de plusieurs images d'un modèle (3) périodique projeté sur un objet (1), qui présente une position de phase connue,
b. dans lequel le premier angle de triangulation ($\theta 1$) est connu,
c. dans lequel chaque image de phase ($\varphi 1$, $\varphi 2$) présente un domaine de clarté (E1, E2), qui dépend entre autres de l'angle de triangulation ($\varphi 1$, $\varphi 2$), dans lequel une attribution d'une valeur de hauteur (h) à une phase de l'image de phase ne peut être réalisée de manière précise dans une plage non ambiguë respective (E1, E2) et
d. dans lequel une représentation de chaque image de phase ($\varphi 1$, $\varphi 2$) s'effectue au moyen d'un facteur de mise à l'échelle (S1,S2) sur les valeurs de hauteur métriques,

**caractérisé en ce que**

e. le deuxième angle de triangulation ($\theta 2$) ne peut être reproduit que de manière approximative et le deuxième facteur de mise à l'échelle (S2) est ainsi erroné,
f. pour chaque pixel (Bi) de la première image de phase $\varphi 1$ un nombre d'ondes (wz) est déterminé en utilisant la deuxième image de phase $\varphi 2$, selon la formule :

$$wz=((r*\varphi 1-\varphi 2)\bmod(2\pi))/2\pi*(1-r),$$

dans laquelle r indique le rapport entre les deux facteurs de mise à l'échelle S1 et S2, et dans laquelle S1 est le premier facteur de mise à l'échelle et S2 le second,
dont la partie entière correspond à l'ordre (n) de la plage non ambiguë (E1), dans lequel se trouve le pixel (Bi) respectif, dans lequel la partie non entière du nombre d'onde (wz) avec une valeur de hauteur (h) augmente de manière constante et ainsi forme une erreur du second facteur de mise à l'échelle erroné (S2)
g. et **en ce qu'**une optimisation du nombre d'ondes (wz) est obtenue en ce sens, qu'au moins la partie non entière pour un échantillon de m pixels (Bi) du nombre d'ondes est rendue minimale par modification du rapport des facteurs de mise à l'échelle (S1, S2) et par modification d'une dérive (9) de la position de phase du modèle (3) entre les deux images de phase ($\varphi 1$, $\varphi 2$),
h. dans lequel le nombre d'onde optimisé est utilisé comme suait :

h.1. pour déterminer une première image de hauteur (H1) de l'objet, **en ce que**, pour chaque pixel (Bi), la somme de l'image de phase ($\varphi 1$) au niveau de ce pixel (Bi) avec 2n fois le nombre d'ondes correspondant (wz) est formée et la somme est multipliée avec le facteur de mise à l'échelle (S1) ou
h.2. pour définir un rapport (r) des facteurs de mise à l'échelle (S1, S2), qui représentent les images de

phase ($\varphi$1 $\varphi$2) sur la valeur de hauteur métrique (h) et d'une dérive (q) entre la position de phase du modèle (3) dans les deux images de phase ($\varphi$1, $\varphi$2) en tant que valeur de départ pour l'optimisation de l'image de hauteur suivante (H) de la mesure en trois dimensions de l'objet suivante ou

h.3. pour créer une seconde image de hauteur métrique (H2) à partir de la seconde image de phase ($\varphi$2).

2. Procédé selon la revendication 1, **caractérisé en ce que**, pour réaliser le procédé de déphasage, un modèle est projeté sur l'objet, qui présente un tracé de luminosité sinusoïdal avec une position de phase connue pouvant être décalée.

3. Procédé selon l'une des revendications 1 ou 2, **caractérisé en ce que** le premier facteur de mise à l'échelle (S1) est connu précisément, qui représente la première image de phase ($\varphi$1) sur la valeur de hauteur métrique (h) et **en ce que** le second facteur de mise à l'échelle (S2) est connu au moins de manière approximative, qui représente la seconde image de phase ($\varphi$1) sur la valeur de hauteur métrique (h).

4. Procédé selon l'une des revendications 1 à 3, **caractérisé en ce que**, lorsque pour la paire d'images de phase enregistrée ($\varphi$1, $\varphi$2) pour au moins deux points, une partie entière du nombre d'onde de ce pixel (Bi) est différente, l'optimisation du nombre d'onde s'effectue en ce sens qu'au moins pour un échantillon de m pixels (Bi), une partie non entière du nombre d'ondes est rendue minimale par modification du rapport (r) des facteurs de mise à l'échelle (S1, S2) via le second facteur de mise à l'échelle (S2) connu au moins de manière approximative et par modification de la dérive (q) du modèle (3) entre les deux images de phase ($\varphi$1, $\varphi$2).

5. Procédé selon l'une des revendications 1 à 4, **caractérisé en ce que**, si pour la paire d'images de phase enregistrée ($\varphi$1, $\varphi$2) les parties entières du nombre d'ondes sont les mêmes pour tous les pixels (Bi), l'optimisation du nombre d'onde s'effectue en ce sens qu'au moins pour un échantillon de m pixels (Bi), la partie non entière du nombre d'ondes est rendue minimale par modification de la dérive (q) entre les deux images de phase ($\varphi$1, $\varphi$2).

6. Procédé selon l'une des revendications 1 à 5, **caractérisé en ce que** des pixels erronés sont exclus pour l'échantillon à m éléments des images de phase ($\varphi$1, $\varphi$2) en fonction de la qualité des données de mesure et d'un procédé de reconnaissance des valeurs aberrantes adapté, dans lequel les pixels de l'échantillon se trouvent notamment au sein d'une distribution attendue pour ce qui est de la partie non entière de la distribution.

7. Procédé selon l'une des revendications 1 à 6, **caractérisé en ce que** l'enregistrement des images pour la première et la seconde image de phase s'effectue par ordre chronologique rapproché.

8. Procédé selon l'une des revendications 1 à 7, **caractérisé en ce que**, pour la création d'une seconde image de hauteur (H2) à partir de la seconde image de phase ($\varphi$2) dans un procédé d'optimisation en utilisant la première image de hauteur (H1) obtenue par l'optimisation, le facteur de mise à l'échelle (S2) connu de façon imprécise, ainsi qu'une modification de positionnement de la caméra entre les images de phase ($\varphi$1) et ($\varphi$2) sont pris en compte, dans lequel, en tant que modification de positionnement de la caméra, un mouvement de rotation et un mouvement de translation sont adoptés.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4575805 A
- US 6040910 A
- EP 0968687 A2
- US 5175601 A
- US 20080239288 A1
- US 20070115484 A1